# FASCICULE DE BREVET EUROPEEN

(11) **EP 2 585 486 B1**
(45) Date de publication et mention de la délivrance du brevet: **14.05.2014**
(21) Numéro de dépôt: 11739109.4
(22) Date de dépôt: 21.06.2011
(51) Int. Cl.: C07K 14/655, A61K 38/04

(54) **NOUVEAUX COMPOSÉS OCTAPEPTIDIQUES ET LEUR UTILISATION THÉRAPEUTIQUE**
NEUE OKTAPEPTID-VERBINDUNGEN UND DEREN THERAPEUTISCHE VERWENDUNG
NEW OCTAPEPTIDIC COMPOUNDS AND THEIR USE IN THERAPY

(30) Priorité: 22.06.2010 EP 10290336
(43) Date de publication de la demande: 01.05.2013
(73) Titulaire: Ipsen Pharma S.a.S., 92100 Boulogne-Billancourt (FR); Commissariat A L'Energie Atomique C.E.A., 75015 Paris (FR); THE CENTRE NATIONAL DE LA RECHERCHE SCIENTIFIQUE (CNRS), 75794 Paris Cedex 16 (FR)
(72) Inventeur: CINTRAT, Jean-Christophe, F-91430 Igny (FR); LIGETI, Melinda, H-1133 Budapest (HU); ROUSSEAU, Bernard, F-92300 Levallois-Perret (FR); ARTZNER, Franck, F-35510 Cesson Sevigne (FR); TARABOUT, Christophe, F-92120 Montrouge (FR); PATERNOSTRE, Marie-Thérèse, F-91370 Verrières le Buisson (FR); FAY, Nicolas, F-78500 Sartrouville (FR); CHERIF-CHEIKH, Roland, E-08860 Castelldefels (Barcelona) (ES); VALERY, Céline, E-08013 Barcelona (ES)
(74) Mandataire: Audonnet, Nathalie
(86) Numéro de dépôt international: PCT/FR2011/000352
(87) Numéro de publication internationale: WO 2011/161332

(56) Documents cités:
- WO-A1-2010/037930
- C VALERY ET AL.: "Molecular origin of the self-assembyl of lanreotide into nanotubes: a molecular approach,", BIOPHYSICAL JOURNAL, vol. 94, no. 5, mai 2008 (2008-05), pages 1782-1795, XP002523806, Biophysical Society ISSN: 0006-3495

## Description

La présente invention concerne de nouveaux composés octapeptidiques. Ces produits ayant une bonne affinité pour certains sous-types de récepteurs de la somatostatine, ils sont particulièrement intéressants pour traiter les états pathologiques ou les maladies dans lesquels un (ou plusieurs) des récepteurs de la somatostatine est (sont) impliqué(s). L'invention concerne également des compositions pharmaceutiques contenant lesdits produits et leur utilisation pour la préparation d'un médicament.

La somatostatine (SST) est un tétradécapeptide cyclique qui a été isolé pour la première fois de l'hypothalamus en tant que substance inhibitrice de l'hormone de croissance (Brazeau P. et al., Science 1973, 179, 77-79). Elle intervient également en tant que neurotransmetteur dans le cerveau (Reisine T. et al., Neuroscience 1995, 67, 777-790 ; Reisine et al., Endocrinology 1995, 16, 427-442). L'hétérogénéité des fonctions biologiques de la somatostatine et les relations structure-activité de ses analogues peptidiques, ont amené la découverte de 5 sous-types de récepteurs liés à la membrane (Yamada et al., Proc. Natl. Acad. Sci. U.S.A, 89, 251-255, 1992 ; Raynor, K. et al, Mol. Pharmacol., 44, 385-392, 1993). Le clonage moléculaire a permis de montrer que la bioactivité de la somatostatine dépend directement de ces cinq sous-types de récepteurs.

Les rôles fonctionnels de ces récepteurs sont actuellement activement étudiés. L'activation préférentielle des sous-types 2 et 5 a été associée à la suppression, dans les adénomes sécrétant ces hormones, de l'hormone de croissance GH (acromégalie), de l'hormone TSH et de la prolactine ; mais le rôle précis de chaque sous-type reste à déterminer.

Parmi les désordres pathologiques associés à la somatostatine (Moreau J.P. et al., Life Sciences, 1987, 40, 419 ; Harris A.G. et al., The European Journal of Medicine, 1993, 2, 97-105), on peut citer par exemple : l'acromégalie, les adénomes hypophysaires, la maladie de Cushing, les gonadotrophinomes et les prolactinomes, les effets secondaires cataboliques des glucocorticoïdes, le diabète, la rétinopathie diabétique, la néphropathie diabétique, l'hyperthyroïdie, le gigantisme, les tumeurs gastroentéropancréatiques endocriniennes dont le syndrôme carcinoïde, le VIPome, l'insulinome, la nésidioblastose, l'hyperinsulinémie, le glucagonome, le gastrinome et le syndrôme de Zollinger-Ellison, le GRFome ainsi que le saignement aigu des varices oesophagiennes, le reflux gastrooesophagien, le reflux gastroduodénal, la pancréatite, les fistules entérocutanées et pancréatiques mais aussi les diarrhées, les diarrhées réfractaires du syndrôme d'immunodépression acquise, la diarrhée chronique sécrétoire, la diarrhée associée avec le syndrome de l'intestin irrité, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénal, la maladie de Crohn, les scléroses systémiques, le dumping syndrome, le syndrome du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et d'autres domaines thérapeutiques comme, par exemple, les céphalées y compris les céphalées associées aux tumeurs hypophysaires, les douleurs, les accès de panique, la chimiothérapie, la cicatrisation des plaies, l'insuffisance rénale résultant d'un retard de croissance, l'obésité et retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis ainsi que la maladie d'Alzheimer. On peut également citer l'ostéoporose.

De nos jours, une attention croissante est portée aux peptides possédant une affinité sur les récepteurs de la somatostatine. Ainsi, le lanréotide a été très étudié pour le traitement des maladies liées à l'hormone de croissance (Cendros JM, Peraire C, Trocôniz IF, Obach R. Pharmacokinetics and population pharmacodynamic analysis of lanreotide Autogel. Metabolism. 2005 Oct, 54(10), 1276-81.)

Des composés octapeptidiques consistant en le lanréotide modifié en position 4 ont été décrits dans la demande de brevet internationale WO-A1-2010037930.

Le besoin de trouver des alternatives aux solutions existantes constitue donc un enjeu majeur. La présente invention s'inscrit dans ce cadre.

La déposante propose donc de nouveaux composés octapeptidiques possédant une bonne affinité sur les récepteurs de la somatostatine.

La présente invention a donc pour objet un composé octapeptidique de formule générale (I)

R-AA¹-cyclo(AA²-Tyr³-D-Trp⁴-AA⁵-Val⁶-Cys⁷)-Thr⁸-NH₂ (I)

dans laquelle AA¹ représente un radical d'acide aminé lié au radical R et à l'acide aminé AA² selon la formule 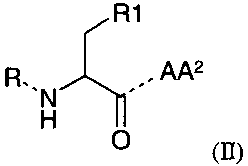 dans laquelle
R1 représente le radical naphthyle éventuellement substitué par un ou plusieurs radicaux alkyle;
R représente un atome d'hydrogène ou un radical alkyle ou acétyle ;
AA² représente un radical d'acide aminé lié aux acides aminés AA¹ et Tyr³ selon la formule 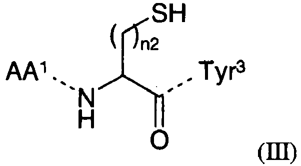 dans laquelle n2 représente un entier de 1 à 2 ;
AA⁵ représente un radical d'acide aminé lié aux acides aminés Trp⁴ et Val⁶ selon la formule 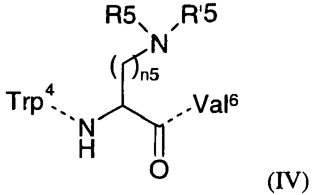 dans laquelle R5 et R'5 représentent indépendamment un atome d'hydrogène ou un radical alkyle ou acétyle, et n5 représente un entier de 1 à 4 ;
étant entendu que
si AA² représente un radical d'acide aminé cystéine, alors
soit AA⁵ représente un radical d'acide aminé lysine et AA¹ ne représente pas un radical d'acide aminé naphthylalanine ;
soit AA¹ représente un radical d'acide aminé naphthylalanine et AA⁵ ne représente pas un radical d'acide aminé lysine ;
soit AA¹ ne représente pas un radical d'acide aminé naphthylalanine et AA⁵ ne représente pas un radical d'acide aminé lysine ;
si AA² ne représente pas un radical d'acide aminé cystéine, alors AA¹ représente un radical d'acide aminé naphthylalanine et AA⁵ représente un radical d'acide aminé lysine,
et étant entendu que tous les acides aminés peuvent être de configuration D ou L,
ou un sel pharmaceutiquement acceptable de ce composé.

Selon la présente invention, on entend par radical d'acide aminé, le radical que forme un acide aminé engagé par ses fonctions amine et acide dans des liaisons peptidiques. Ainsi, un radical d'acide aminé ayant X comme chaîne latérale aura pour radical le radical de formule -NH-CH(X)-C(O)-.

Selon la présente invention, les acides aminés représentés par leur code à trois lettres dans une formule générale, ou en tant que tels, ou bien encore en tant que radicaux, peuvent être de configuration D ou L, si rien n'est précisé.

Par ailleurs, selon la présente invention et conformément à la convention, la dénomination des peptides exemplifiés par leur séquence d'acides aminés représentés par leur code à trois lettres, mentionne les acides aminés configuration L sans rien préciser tandis que les acides aminés D sont indiqués explicitement par la lettre D précédant le code à trois lettre de l'acide aminé considéré.

Au sens de la présente invention, on entend par alkyle, lorsqu'il n'est pas donné plus de précision, un radical alkyle linéaire ou ramifié comptant de 1 à 6 atomes de carbone, tels que méthyle, éthyle, propyle, isopropyle, butyle, isobutyle, tert-butyle, pentyle, ou hexyle, et de préférence de 1 à 4 atomes de carbone.

Selon la présente invention, l'expression sel pharmaceutiquement acceptable définit des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Pour d'autres exemples de sels pharmaceutiquement acceptables, on peut se référer à "Salt selection for basic drugs", Int. J. Pharm. (1986), 33, 201-217.

Selon la présente invention, les acides aminés représentent les acides aminés connus de l'homme de l'art de configuration D ou L, représentés ici selon leur nomenclature habituelle et les analogues synthétiques modifiés sur les chaînes latérales desdits acides aminés parmi lesquels :
D-2-Nal1, Ac-D-2-Nal1, CH₃-D-2-Nal1 représentent une alanine substituée en β par un radical naphthyle respectivement non substituée et substituée sur sa fonction amine par un radical acétyle ou méthyle sur sa position 1 ;
Hcy représente une homocystéine, c'est-à-dire une cystéine dont la chaîne latérale est allongée d'un chaînon méthylène ;
Orn représente une ornithine, c'est-à-dire une lysine dont la chaîne latérale est raccourcie d'un chaînon méthylène ;
Dab représente l'acide diaminobutyrique, c'est-à-dire une lysine dont la chaîne latérale est raccourcie de deux chaînons méthylène ;
Dap représente l'acide diaminopropionique, c'est-à-dire une lysine dont la chaîne latérale est raccourcie de trois chaînons méthylène ;
Lys(Ac) et (Me)₂Lys représentent une lysine substituée sur la fonction amine de sa chaîne latérale par respectivement un ou plusieurs radicaux acétyle ou méthyle.
De préférence, n5 représente un entier de 1 à 4, et de manière encore plus préférentielle, n5 représente un entier de 3 à 4.
De préférence, AA² représente un radical d'acide aminé cystéine, c'est-à-dire n2 est égal à 1.
De préférence, AA² ne représente pas un radical d'acide aminé cystéine, c'est-à-dire n2 est égal à 2.

De préférence, AA¹ ne représente pas un radical d'acide aminé naphthylalanine, c'est-à-dire R représente un radical alkyle ou acétyle et AA⁵ représente un radical d'acide aminé lysine, c'est-à-dire n5 est égal à 4 et R5 et R'5 représentent des atomes d'hydrogène.

De préférence, AA¹ représente un radical d'acide aminé naphthylalanine, c'est-à-dire R représente un atome d'hydrogène, et AA⁵ ne représente pas un radical d'acide aminé lysine, c'est-à-dire soit n5 est un entier de 1 à 3 soit n5 est égal à 4 et R5 et R'5 représentent un radical alkyle ou acétyle.

De préférence, AA¹ ne représente pas un radical d'acide aminé naphthylalanine, c'est-à-dire R représente un radical alkyle ou acétyle, et AA⁵ ne représente pas un radical d'acide aminé lysine c'est-à-dire soit n5 est un entier de 1 à 3 soit n5 est égal à 4 et R5 et R'5 représentent un radical alkyle ou acétyle.

De préférence, AA¹ représente le radical d'acide aminé choisi parmi 2-Nal, Ac-2-Nal et CH₃-2-Nal. De manière préférée, AA¹ est de configuration D.

De préférence, AA² représente le radical d'acide aminé choisi parmi Cys et Hcy.

De préférence, AA⁵ représente le radical d'acide aminé choisi parmi Lys, Lys(Ac), Orn, (CH₃)₂Lys, Dab et Dap.

De préférence, le composé octapeptidique de formule générale (I) est choisi parmi :

H-D-2-Nal¹-cyclo(Cys²-Tyr³-D-Trp⁴-Lys(Ac)⁵-Val⁶-Cys⁷)-Thr⁸-NH₂

Ac-D-2-Nal¹-cyclo(Cys²-Tyr³-D-Trp⁴-Lys(Ac)⁵-Val⁶-Cys⁷)-Thr⁸-NH₂

H-D-2-Nal¹-cyclo(Cys²-Tyr³-D-Trp⁴-Orn⁵-Val⁶-Cys⁷)-Thr⁸-NH₂

H-D-2-Nal¹-cyclo(Cys²-Tyr³-D-Trp⁴-(Me)₂Lys⁵-Val⁶-Cys⁷)-Thr⁸-NH₂

H-O-2-Nal¹-cyclo(Hcy²-Tyr³-D-Trp⁴-Lys⁵-Val⁶-Cys⁷)-Thr⁸-NH₂

Ac-D-2-Nal¹-cyclo(Cys²-Tyr³-D-Trp⁴-Lys⁵-Val⁶-Cys⁷)-Thr⁸-NH₂

CH₃-D-2-Nal¹-cyclo(Cys²-Tyr³-D-Trp⁴-Lys⁵-Val⁶-Cys⁷)-Thr⁸-NH₂

H-D-2-Nal¹-cyclo(Cys²-Tyr³-D-Trp⁴-Dab⁵-Val⁶-Cys⁷)-Thr⁸-NH₂

H-D-2-Nal¹-cyclo(Cys²-Tyr³-D-Trp⁴-Dap⁵-Val⁶-Cys⁷)-Thr⁸-NH₂

ou un sel pharmaceutiquement acceptable de ce composé.

De manière plus préférentielle, le composé octapeptidique de formule générale (I) est choisi parmi :

H-D-2-Nal¹-cyclo(Cys²-Tyr³-D-Trp⁴-Orn⁵-Val⁶-Cys⁷)-Thr⁸-NH₂ ;

H-D-2-Nal¹-cyclo(Cys²-Tyr³-D-Trp⁴-(Me)₂Lys⁵-Val⁶-Cys⁷)-Thr⁸-NH₂ ;

H-D-2-Nal¹-cyclo(Hcy²-Tyr³-D-Trp⁴-Lys⁵-Val⁶-Cys⁷)-Thr⁸-NH₂ ; et

Ac-D-2-Nal¹-cyclo(Cys²-Tyr³-D-Trp⁴-Lys⁵-Val⁶-Dys⁷)-Thr⁸-NH₂ ;

ou un sel pharmaceutiquement acceptable de ce composé.

De manière encore plus préférentielle, le composé octapeptidique de formule générale (I) est : H-D-2-Nal¹-cyclo(Hcy²-Tyr³-D-Trp⁴-Lys⁵-Val⁶-Cys⁷)-Thr⁸-NH₂ ou un sel pharmaceutiquement acceptable de ce composé.

L'invention a également pour objet un médicament comprenant un composé selon l'invention tel que défini précédemment.

L'invention a également pour objet une composition pharmaceutique comprenant un composé selon l'invention tel que défini précédemment et plus particulièrement lorsque le composé est utilisé à titre de principe actif.

L'invention a également pour objet une composition thérapeutique comprenant un composé de formule générale (I) tel que défini précédemment, en tant que principe actif en association avec au moins un excipient pharmaceutiquement acceptable.

L'invention a également pour objet une utilisation d'un composé octapeptidique de formule générale (I) tel que défini précédemment pour fabriquer un médicament.

L'invention a également pour objet une utilisation telle que définie ci-dessus dans laquelle le médicament est destiné à traiter une pathologie choisie parmi les maladies liées à l'hormone de croissance.

L'invention concerne enfin l'utilisation d'un composé tel que défini précédemment pour fabriquer un médicament ; et préférentiellement un médicament destiné à traiter les pathologies dans lesquelles un (ou plusieurs) récepteur(s) à la somatostatine est (sont) impliqué(s), telles que l'acromégalie, le traitement des tumeurs neuroendocrines, la rétinopathie diabétique, le traitement des vaisseaux, des articulation et de la peau ; et de manière préférentielle l'acromégalie ou le traitement des tumeurs neuroendocrines.

La composition thérapeutique selon l'invention peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

La composition thérapeutique selon l'invention peut aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'une composition selon l'invention pourra se faire par voie topique, orale, parentérale, par injection intramusculaire, sous-cutanée etc.

A moins qu'ils ne soient définis d'une autre manière, tous les termes techniques et scientifiques utilisés dans la présente demande ont la même signification que celle couramment comprise par un spécialiste ordinaire du domaine auquel appartient l'invention.

La partie expérimentale suivante est présentée pour illustrer les procédures ci-dessus et ne doit en aucun cas être considérée comme une limite à la portée de l'invention.

### PARTIE EXPERIMENTALE

### 1. Description des synthèses

### 1.1 Matériel utilisé

### HPLC-MS :

Le système est de marque Waters (2525) avec dégazeur en ligne et système d'injection automatisé (2767). L'élution consiste en un gradient d'eau et d'acétonitrile, avec 0,1 % d'acide formique. La détection des espèces éluées se fait par une barrette de diodes (2996), un détecteur évaporatif à diffusion de lumière (DEDL) et un spectromètre de masse (voir ci-après). La colonne est de type phase inverse, greffée C18, modèle X-Bridge 100 x 4.6 mm avec une taille de particules de 3,5 µm et une taille de pores de 13,5 nm. Le débit est réglé à 1 mL min-1 et le volume d'injection à 20 µl.

Le spectromètre de masse est un Micromass ZQ de marque Waters. L'ionisation se fait par électrospray, avec une température de la source de 120° C et une tension de cône de 20 V. L'échantillon est introduit de façon continue à 0,3 mL min-1. L'analyseur est de type quadripôle (modèle ZQ2000). Les spectres sont enregistrés à l'aide du logiciel Mass Lynx 4.0 dans le domaine de m/z 100-1000 pour les molécules organiques et 100-2000 pour les peptides.

### HPLC-préparative :

Deux systèmes sont utilisés pour la purification de peptides. Le système précédemment décrit équipé d'une colonne type phase inverse, greffée C18, modèle X-Bridge 150 x 19 mm avec une taille de particules de 5 µm et une taille de pores de 13,5 nm. Le débit est de 17 mL min-1. Le deuxième système est un Waters 2545 similaire au précédent, non équipé d'un spectromètre de masse. La colonne est une Thermo Hypurity, de type phase inverse (greffée C18) de taille 21,2 x 250 mm. Elle est éluée par un mélange d'eau et d'acétonitrile avec 0,1 % de TFA à un débit de 20 mL min-1. Les deux systèmes de HPLC préparative sont utilisés en mode isocratique après détermination des conditions optimales.

### Analyses RMN :

Les analyses par Résonance Magnétique Nucléaire sont réalisées sur un spectromètre Bruker Advance 400 Ultrashield. Les fréquences d'analyse sont de 400 MHz pour le protonet 100 MHz pour le carbone 13. Les spectres sont enregistrés à température ambiante, les déplacements chimiques sont exprimés en ppm et les constantes de couplage en Hz. La multiplicité est donnée de la façon suivante : s = singulet, Is = large singulet, d = doublet, Id = large doublet, dd = doublet de doublets, ddd = doublet de doublets dédoublés, t = triplet, It= large triplet, q = quadruplet, qd = quadruplet dédoublé, m = multiplet.

### Analyses HRMS :

Les mesures de masse exacte ont été effectuées sur un spectromètre de masse à temps de vol (LCT de Micromass®, UK), muni d'une source electrospray (source Z-spray) en mode positif. La référence externe permettant la mesure de masse exacte est introduite en parallèle de l'échantillon et de façon continue (configuration Lockspray™). Celle ici utilisée est la Leucine Enképhaline qui donne un ion [M+Na]+ à m/z = 578,2591. La résolution de cet appareil est de 6500 et les résultats sont donnés avec un écart par rapport à la masse théorique inférieur à 5 mDa. L'appareil est piloté par le logiciel Masslynx 4.0® L'échantillon solubilisé dans l'eau est injecté dans un flux 50 % eau-50 % méthanol via une HPLC munie d'un passeur automatique d'échantillons (Alliance 2795 de Waters®, UK) à un débit de 200 µl min-1. Le volume d'injection est de 10 µl. La tension du capillaire est de 2800 V. La tension de cône est de 40 V. La température de la source est de 120° C. La température de désolvatation est de 250° C. Le débit du gaz de désolvatation (azote) est de 500 l h-1. Le débit du gaz de cône (azote) est de 20 l h-1. TDC Stop : 100 mV

### Spectrométrie IR :

Les spectres infrarouges des peptides sont enregistrés par réflexion totale atténuée et par transformée de Fourier. L'appareil est un Bruker IFS 66 équipé d'un module 45°N Znse ATR, purgé en continu avec de l'azote. 10 µL de solution sont déposés sur le cristal et trente balayages sont moyennés à une résolution de 4 cm-1. Le signal de l'eau est soustrait du spectre brut grâce au logiciel OPUS 4.2.

### Lyophilisateur :

Le lyophilisateur utilisé est un Christ Alpha 2-4 LD plus connecté à une pompe à palette permettant d'atteindre des vides d'environ 15 µbar. Les échantillons aqueux sont solidifiés dans l'azote liquide avant d'être connectés à cet appareil.

### Microscope :

MET de type Microscope Phillips CM-20 opérant à 200 kV, et MEB de type Léo-Gémini, field emission gun (canon à émission de champ).

### Réactifs utilisés

La résine de synthèse peptidique est obtenue chez Novabiochem, division de Merk Bioscience (Schwalbach, Allemagne). La résine échangeuse d'ions provient des laboratoires Bio-Rad (Hercules, Etats-Unis).

L'eau utilisée est doublement désionisée par utilisation d'un système échangeur Milli-Q Plus de Millipore (Billerica, Etats-Unis). Les solvants pour les synthèses et pour les purifications sont achetés chez Aldrich et VWR (West Chester, Etats-Unis) et, sauf mention contraire, sont utilisés sans purification.

Les acides aminés sont achetés chez Bachem (Weil am Rhein, Allemagne), Fluka (Buchs, Suisse), Acros Organics (Geel, Belgique) et NeoMPS (Strasbourg, France).

### Acides aminés précurseurs disponibles commercialement :

Les acides aminés Fmoc-Lys(Ac)-OH, Acetyl-β-(2-naphthyl)-D-alanine, Fmoc-Orn(Boc)-OH, Fmoc-Homocys(Trt)-OH, Fmoc-Dab(Boc)-OH, Fmoc-Dap(Boc)-OH sont disponibles commercialement.

### Synthèse de l'acide aminé N-Méthyl-Boc-D-2-Nal-OH

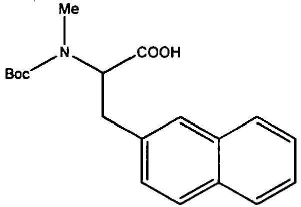

À une suspension de 2,4 g (60 mmol) de NaH (60 % dans l'huile minérale) dans 80 mL de THF anhydre placée à 0° C sont ajoutés 3,2 g de (10 mmol) de Boc-D-2-Nal-OH dissous dans 20 mL de THF anhydre. Le milieu réactionnel est agité 15 minutes à 0° C puis 10 mL (160 mmol) de Mel sont ajoutés goutte à goutte. La réaction est ensuite poursuivie pendant 4 h à 0° C, 5 mL de Mel sont alors rajoutés, le milieu réactionnel est ensuite ramené à température ambiante puis laissé sous agitation toute la nuit. Le milieu réactionnel est neutralisé à 0° C par 20 mL d'une solution saturée de NH₄Cl puis le THF et le Mel sont évaporés à l'évaporateur rotatif. 100 mL d'hexane sont alors ajoutés, la phase aqueuse est récupérée, lavée 2 fois par 100 mL d'hexane. 100 mL d'acétate d'éthyle sont ajoutés et la phase aqueuse est acidifiée (pH 2), la phase aqueuse est ensuite extraite par 3 fois 100 mL d'acétate d'éthyle. Les phases organiques sont ensuite réunies, séchées sur Na₂SO₄, filtrées puis évaporées. Le produit attendu est ensuite cristallisé dans un mélange hexane/acétate d'éthyle (1,82 g, 55 %).

¹H-RMN (CD₃OD) : δ 1,22 (S, 6H, tBu), 1,27 (s, 3H, tBu), 2,72 (s, 3H, Me), 3,42-3,49 (m, 1 H), 4,78 (dd, 1H, J = 4,1, J = 11,5), 4,97 (dd, 1H, J = 4,7, J = 11,5), 7,37-7,46 (m, 3H), 7,67 (s, 1 H), 7,76-7,82 (m, 3H).

MS (ESI) : m/z 330.0 [M+H]+

### Synthèse de l'acide aminé Fmoc Nε,ε diméthyl lysine (d'après Int J. Peptide Res. Ther. (2006), 12, 187-193)

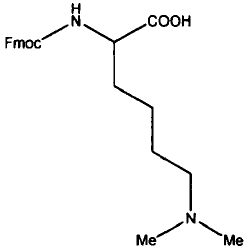

203 mg (0,5 mmol) de chlorhydrate de Fmoc-Lys-OH sont dissous dans 10 mL d'éthanol et la solution est placée à 0° C. 86 µL (1,1 mmol) de formaldéhyde (solution à 37 %) sont ajoutés et l'ensemble est maintenu à 0° C pendant 15 minutes. 95 mg (1,5 mmol) de NaBH₃CN sont alors ajoutés et la réaction est poursuivie pendant 15 minutes à 0° C avant de rajouter 86 µL de formaldéhyde et 95 mg de NaBH₃CN et de laisser la réaction se poursuivre pendant 4 heures à 0° C. 10 mL d'une solution d'HCl 1 N sont ajoutés, le précipité obtenu est filtré, la solution est ensuite portée à sec et le composé obtenu est purifié par chromatographie (éluant MeOH/H₂0 95/5) pour conduire à 110 mg de composé attendu (avec une très faible quantité de lysine triméthylée).

¹H-RMN (CD₃OD) : δ 1,4-1,9 (m, 6H); 2,7 (s, 6H); 2,91 (t, J = 7,7, 2H); 4,02 (dd, J = 5,3, J = 7,1, 1 H); 4,19 (t, J = 6,8, 1 H); 4,22-4,35 (m, 2H); 7,28- 7,32 (m, 2H) ; 7,38 (t, J = 7,2, 2H); 7,63-7,72 (m, 2H); 7,79 (d, J = 7,5, 2H).

MS (ESI) m/z : 397,0 [M+H]+

### 1.3 Préparation des composés octapeptidiques

### 1.3.1 Procédure générale pour la synthèse de peptides

La synthèse comporte 4 étapes principales comme le montre le schéma 1 :

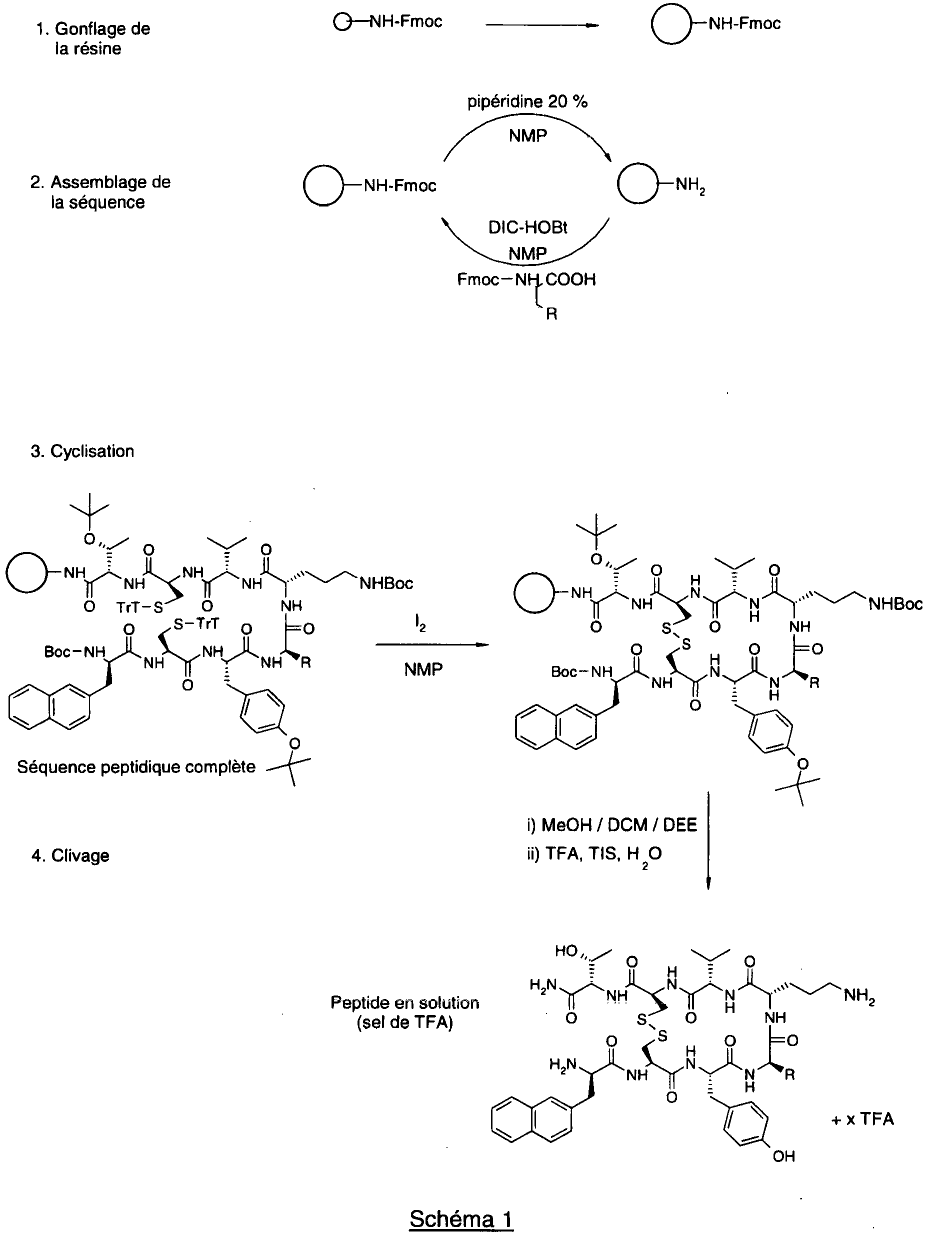

### 1/ Mouillage de la résine :

La résine 4-(2',4'-diméthoxyphényl-fluorènylméthoxycarbonyl-aminométhyl)-phénoxyacétamido-norleucyl-(4-méthyl)-benzhydrylamine - base polystyrène - divinylbenzène (Rink Amide MBHA) est introduite dans une seringue munie d'un verre fritté, d'un robinet à une extrémité et d'un bouchon à l'autre. On la remplit avec de la NMP et le mélange est mis sous agitation douce pendant 1 h. Le solvant est ensuite éliminé par filtration.

### 2/ Couplage des acides aminés :

Les acides aminés sont couplés les uns aux autres dans l'ordre souhaité au moyen d'une réaction de couplage. L'acide aminé (2 eq) est introduit avec le 1-hydroxybenzotriazole (HOBt, 2,2 eq) et le *N*,*N*'-Diisopropylcarbodiimide (DIC, 2,2 eq) dans la N-méthylpyrrolidinone (NMP, 5 mL/g de résine) dans un tube à essai et agité quelques minutes. Il est ensuite mis en présence de la résine dans son récipient. Le mélange réactionnel est agité pendant 1 h 30 puis filtré. La technique du double couplage est utilisée : le mélange réactionnel est filtré quand la réaction est environ à 50 % d'avancement, et des réactifs frais sont réintroduits, afin d'optimiser la vitesse de réaction et la pureté du produit final. La deuxième étape consiste à déprotéger le nouvel acide aminé introduit, afin de permettre un nouveau couplage. La déprotection est réalisée par trois traitements par de la pipéridine dans la NMP (20 % v/v), 5 mUg de résine suivis par trois lavages à la NMP (10 mUg de résine). Afin de suivre la réaction, 5 µl du filtrat correspondant au premier traitement, puis 10 µl des deux suivants, ainsi que le premier lavage, soit 4 échantillons, sont introduits dans 2 mL de pipéridine avant mesure de l'absorbance UV à 290 nm. Entre chaque étape, on procède à trois lavages de la résine par la NMP (10 mUg de résine). Cette étape d'assemblage consiste donc en deux réactions : la réaction de couplage d'acides aminés et la réaction de déprotection du groupement Fmoc, à réaliser itérativement jusqu'à achèvement de la séquence peptidique.

### 3/ Formation du pont disulfure :

Une fois la séquence assemblée, le peptide doit être cyclisé par formation du pont disulfure. Le pont disulfure est formé par trois traitements de diiode 1 eq dans la NMP (5 mUg de résine) pendant respectivement 2 min, 3 min et 5 min. La résine est ensuite lavée 5 fois par du DCM et 5 fois par de la NMP afin d'éliminer l'excès d'iode retenu dans la résine (10 mUg de résine).

### 4/ Clivage :

La résine doit être préparée au clivage par deux lavages à la NMP, deux lavages au méthanol (MeOH), deux lavages au dichlorométhane (DCM) et deux lavages à l'éther diéthylique (DEE) (10 mL par gramme de résine). La résine est ensuite mise sous vide pendant une journée. Le clivage se fait dans un ballon en verre muni d'un agitateur magnétique. Le mélange réactionnel est formé d'acide trifluoroacétique (TFA, 10 mL/g de résine) ainsi que de triisopropylsilane (TIS) et de l'eau (3 % et 2 % v/v). La réaction est agitée 4 h à température ambiante. Le milieu est ensuite filtré sur verre fritté et le solide lavé deux fois par le TFA. Le filtrat est ensuite évaporé pour obtenir un liquide blanc très épais. Celui-ci est dissous dans un mélange eau-acétonitrile 1:1 afin d'être lyophilisé. Après cette étape, le peptide se présente sous forme de sel de trifuoroacétate.

### 1.3.2 Purification

La purification est réalisée par chromatographie liquide haute performance (HPLC) préparative. La phase stationnaire est dite "inverse" car greffée avec des chaînes alkyles C₁₈. La phase mobile est constituée d'un mélange fixé (isocratique) d'eau et d'acétonitrile avec 0,1 % de TFA ou 1 % d'acide formique servant à neutraliser les silanols non greffés résiduels pouvant exister sur la phase stationnaire.

Le peptide doit être dissous dans un mélange eau-acétonitrile pour être injecté en HPLC préparative. Une étude de solubilité est d'abord réalisée sur une petite quantité. Elle permet d'établir le pourcentage d'acétonitrile optimal et la quantité maximale de peptide. Un pourcentage d'acétonitrile le plus faible possible avec une concentration de peptide très élevée et une solution résultante limpide constituent des conditions idéales.

Après purification, les fractions contenant le peptide purifié sont rassemblées et évaporées sous vide. Le peptide pur est alors récupéré dans de grandes quantités de solvant à évaporer avant de procéder à une lyophilisation. Le peptide se présente en général sous forme de sel de trifluoroacétate devant être échangé par un acétate avant analyse physico-chimique.

### 1.3.3 Echange d'ions

L'échange est réalisé sur une résine de type échangeuse d'anions forts (AG1-X8 Biorad). 245 mg de cette résine sont d'abord lavés par trois fois 10 mL d'acide acétique 1,6 N puis par trois fois 10 mL d'acide acétique 0,16 M. 20 mg de peptide en sel de TFA sont alors introduits dans 4 mL d'eau et le récipient est agité de manière rotative pendant 1 h. Le liquide est alors filtré et la résine lavée par deux fois 1 mL d'eau distillée. Les fractions sont rassemblées puis lyophilisées.

### 1.4 Exemples

Les produits ont été caractérisés selon les méthodes classiques connues de l'homme de l'art décrites précédemment.

### Exemple 1 : H-D-2-Nal¹-cyclo(Cys²-Tyr³-D-Trp⁴-Orn⁵-Val⁶-Cys⁷)-Thr⁸-NH₂

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Orn(Boc)-OH puis Fmoc-D-Trp-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-Nal¹-cyclo(Cys²-Tyr³-D-Trp⁴-Om⁵-Val⁶-Cys⁷)-Thr⁸-NH₂.

HPLC : tr = 8,65 min

HRMS (H₂O) m/z = 1082.46102 [M+H]⁺ (calc. 1082.45866)

¹H NMR (400 Mhz, D₂O): δ 0,3-0,44 (m, 1 H); 0,49-0,64 (m, 1 H); 0,76 (d, J = 8, 3H); 0,78 (d, J = 7, 3H); 1-1,12 (m, 1 H); 1,06 (d, J = 6,5, 3H); 1,45-1,57 (m, 1 H); 1,94-2,07 (m, 1 H); 2,31 (dd, J = 4, J = 14,7, 1 H); 2,36-2,5 (m, 3H); 2,54-2,68 (m, 2H); 2,77 (d, J = 7,5, 2H); 2,82-2,95 (m, 2H); 3,19 (dd, J = 9, J = 13,6, 1 H); 3,33 (dd, J = 6, J = 13,6, 1 H); 3,74 (dd, J = 4, J = 10,8, 1 H); 3,82 (d, J = 9,6, 1 H); 4,04-4,14 (m, 2H); 4,17 (d, J = 3,8, 1 H); 4,24 (dd, J = 6, J = 8,7, 1 H); 4,49 (t, J = 7,5, 1 H); 4,61-4,76 (m, 3H); 6,69 (d, J = 8,5, 2H); 6,97 (d, J = 8,5, 2H); 6,97-7,05 (m, 2H); 7,1 (bt, J = 7,5, 1H); 7,29-7,45 (m, 4H); 7,65 (bs, 1 H); 7,72-7,81 (m, 3H).

### Exemple 2 : H-D-2-Nal¹-cyclo(Cys²-Tyr³-D-Trp⁴-Nε,ε-dimethyl-Lys⁵-Val⁶-Cys⁷)-Thr⁸-NH₂ (H-D-2-Nal¹-cyclo(Cys²-Tyr³-D-Trp⁴-(Me)₂Lys⁵-Val⁶-Cys⁷)-Thr⁸-NH₂)

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-(Me)₂Lys-OH puis Fmoc-D-Trp-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-Nal¹-cyclo(Cys²-Tyr³-D-Trp⁴-Nε,ε-dirnethyl-Lys⁵-Val⁶-Cys⁷)-Thr⁸-NH₂.

HPLC : tr = 10.48 min

HRMS (H₂O) m/z = 1124.50620 [M+H]⁺ (calc. 1124.50561)

¹H NMR (400 Mhz, D₂O): δ -0,03-0,1 (m, 1H); 0,17-0,31 (m, 1H); 0,76 (d, J = 7,2, 3H); 0,78 (d, J = 7, 3H); 0,97-1,18 (m, 3H); 1,07 (d, J = 6,3, 3H); 1,38-1,51 (m, 1H); 1,95-2,07 (m, 1 H); 2,25-2,33 (m, 1 H); 2,36-2,69 (m, 5H); 2,63 (s, 6H); 2,78 (bd, J = 7,3, 2H); 2,78-2,95 (m, 2H); 3,13 (dd, J = 9,5, J = 12,8, 1 H); 3,27 (dd, J = 5,9, J = 13,3, 1 H); 3,7 (dd, J = 2,1, J = 9,9, 1H); 3,81 (d, J = 9,6, 1H); 4,05-4,16 (m, 3H); 4,18 (d, J = 3,6, 1 H); 4,5 (t, J = 7,5, 1 H); 4,64-4,73 (m, 3H); 6,7 (d, J = 8,4, 2H); 6,95-7,05 (m, 4H); 7,1 (t, J = 7,5, 1 H); 7,33 (t, J = 8,4, 2H); 7,36-7,45 (m, 3H); 7,63 (bs, 1 H); 7,70-7,75 (m, 1 H); 7,77 (d, J = 8,6, 1 H).

### Exemple 3 : N-Methyl-D-2-Nal¹-cyclo(Cys²-Tyr³-D-Trp⁴-Lys⁵-Val⁶-Cys⁷)-Thr⁸-NH₂ (CH₃-D-2-Nal¹-cyclo(Cys²-Tyr³-D-Trp⁴-Lys⁵-Val⁶-Cys⁷)-Thr⁸-NH₂)

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-D-Trp-OH puis Fmoc-L-Tyr(tBu)-OH puis Fmoc-L-Cys(Trt)-OH et enfin Boc-N-Methyl-D-(2-naphthyl)-Ala-OH.

Le composé est cyclisé par la formation d'un pont disulfure, puis clivé de la résine, purifié, et enfin obtenu sous forme d'acétate selon le mode opératoire décrit précédemment pour conduire au composé N-Methyl-D-2-Nal¹-cyclo(Cys²-Tyr³-D-Trp⁴-Lys⁵-Val⁶-Cys⁷)-Thr⁸-NH₂.

HPLC : tr = 8.83 min

HRMS (H₂O) m/z = 1110.48991 [M+H]⁺ (calc. 1110.48996)

¹H NMR (400 Mhz, D₂O): δ -0,05-0,1 (m, 1H); 0,2-0,32 (m, 1 H); 0,76 (d, J = 6,4, 3H); 0,78 (d, J = 6,4, 3H); 0,94-1,16 (m, 3H); 1,08 (d, J = 6,4, 3H); 1,35-1,48 (m, 1 H); 1,96-2,09 (m, 1 H); 2,16 (dd, J = 3,8, J = 14,8, 1 H); 2,35 (dd, J = 9,3, J = 14,8, 1H); 2,39-2,64 (m, 4H); 2,52 (s, 3H); 2,69-2,96 (m, 4H); 3,16 (dd, J = 10,2, J = 13,3, 1H); 3,38 (dd, J = 5, J = 13,3, 1 H); 3,65 (dd, J = 3,5, J = 11, 1 H); 3,78 (d, J = 9,8, 1 H); 4,06-4,19 (m, 3H); 4,22 (d, J = 4, 1 H); 4,5 (t, J = 7,6, 1 H); 4,55-4,75 (m, 3H); 6,68 (d, J = 8,5, 2H); 6,95-7,03 (m, 3H); 7,09 (t, J = 7,2, 1 H); 7,29 (dd, J = 1,3, J = 8,4, 1 H); 7,33 (d, J = 8,2, 1 H); 7,37-7,44 (m, 3H); 7,62 (bs, 1 H); 7,68-7,73 (m, 1 H); 7,75-7,8 (m, 2H).

### Exemple 4 : H-D-2-Nal¹-cyclo(Cys²-Tyr³-D-Trp⁴-Dab⁵-Val⁶-Cys⁷)-Thr⁸-NH₂

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-Dab(Boc)-OH puis Fmoc-D-Trp-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-Nal¹-cyclo(Cys²-Tyr³-D-Trp⁴-Dab⁵-Val⁶-Cys⁷)-Thr⁸-NH₂.

HPLC : tr = 8.68 min

HRMS (H₂O) m/z = 1068.44355 [M+H]⁺ (calc. 1068.44301)

¹H NMR (400 Mhz, D₂O): 0,76 (d, J = 6,7, 3H); 0,77 (d, J = 6,7, 3H); 1,06 (d, J = 6,4, 3H); 1,21-1,36 (m, 1H); 1,37-1,47 (m, 1H); 1,78-1,9 (m, 2H); 1,96-2,08 (m, 1H); 2,34 (dd, J = 3,8, J = 14,8, 1 H); 2,46 (dd, J = 9,7, J = 14,8, 1 H); 2,54-2,68 (m, 2H); 2,78 (d, J = 7,4, 2H); 2,91 (d, J = 8,3, 2H); 3,16 (dd, J = 9,2, J = 13,5, 1 H); 3,29 (dd, J = 5,6, J = 13,5, 1 H); 3,82 (d, J = 9,8, 1 H); 3,86 (dd, J = 3,2, J = 11, 1 H); 4,03-4,13 (m, 2H); 4,13-4,21 (m, 2H); 4,49 (t, J = 7,4, 1 H); 4,63-4,78 (m, 3H); 6,69 (d, J = 8,4, 2H); 6,97 (d, J = 8,5, 2H); 6,99-7,06 (m, 2H); 7,11 (t, J = 7,5, 1 H); 7,35 (dd, J = 1, J = 8,5, 1 H); 7,37 (d, J = 8,2, 1 H); 7,38-7,44 (m, 2H); 7,65 (bs, 1 H); 7,70-7,82 (m, 3H).

### Exemple 5 : H-D-2-Nal¹-cyclo(Cys²-Tyr³-D-Trp⁴-Dap⁵-Val⁶-Cys7)-Thr⁸-NH₂

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-Dap(Boc)-OH puis Fmoc-D-Trp-OH puis Fmoc-L-Tyr(tBu)-OH puis Fmoc-L-Cys(Trt)-OH et enfin Boc-β-(2-naphthyl)-D-Ala-OH,

Le composé est cyclisé par la formation d'un pont disulfure, puis clivé de la résine, purifié, et enfin obtenu sous forme d'acétate selon le mode opératoire décrit précédemment pour conduire au composé H-D-2-Nal¹-cyclo(Cys²-Tyr³-D-Trp⁴-Dap⁵-Val⁶-Cys⁷)-Thr⁸-NH₂.

HPLC : tr = 8,65 min

HRMS (H₂O) m/z = 1054,42780 [M+H]⁺ (calc, 1054,42736)

¹H NMR (400 Mhz, D₂O): 0,76 (d, J = 6,7, 3H); 0,77 (d, J = 6,7, 3H); 1,06 (d, J = 6,4, 3H); 1,21-1,36 (m, 1 H); 1,37-1,47 (m, 1 H); 1,78-1,9 (m, 2H); 1,96-2,08 (m, 1 H); 2,34 (dd, J = 3,8, J = 14,8, 1 H); 2,46 (dd, J = 9,7, J = 14,8, 1 H); 2,54-2,68 (m, 2H); 2,78 (d, J = 7,4, 2H); 2,91 (d, J = 8,3, 2H); 3,16 (dd, J = 9,2, J = 13,5, 1 H); 3,29 (dd, J = 5,6, J = 13,5, 1 H); 3,82 (d, J = 9,8, 1H); 3,86 (dd, J = 3,2, J = 11, 1 H); 4,03-4,13 (m, 2H); 4,13-4,21 (m, 2H); 4,49 (t, J = 7,4, 1 H); 4,63-4,78 (m, 3H); 6,69 (d, J = 8,4, 2H); 6,97 (d, J = 8,5, 2H); 6,99-7,06 (m, 2H); 7,11 (t, J = 7,5, 1 H); 7,35 (dd, J = 1, J = 8,5, 1 H); 7,37 (d, J = 8,2, 1H); 7,38-7,44 (m, 2H); 7,65 (bs, 1 H); 7,70-7,82 (m, 3H).

### Exemple 6 : H-D-2-Nal¹-cyclo(Cys²-Tyr³-D-Trp⁴-N-Ac-Lys⁵-Val⁶-Cys⁷)-Thr⁸-NH₂ (H-D-2-Nal¹-cyclo(Cys²-Tyr³-D-Trp⁴-Lys(Ac)⁵-Val⁶-Cys⁷)-Thr⁸-NH₂)

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-Lys(Ac)-OH puis Fmoc-D-Trp-OH puis Fmoc-L-Tyr(tBu)-OH puis Fmoc-L-Cys(Trt)-OH et enfin Boc-β-(2-naphthyl)-D-Ala-OH,

Le composé est cyclisé par la formation d'un pont disulfure, puis clivé de la résine, purifié, et enfin obtenu sous forme d'acétate selon le mode opératoire décrit précédemment pour conduire au composé H-D-2-Nal¹-cyclo(Cys²-Tyr³-D-Trp⁴-N-Ac-Lys⁵-Val⁶-Cys⁷)-Thr⁸-NH₂.

HPLC : tr = 10,58 min

HRMS (H₂O) m/z = 1138,4886 [M+H]⁺ (calc, 1138,4854)

¹H NMR (400 Mhz, D₂O/CD₃CN): δ 0,45-0,59 (m, 1 H); 0,62-0,73 (m, 1 H); 1,10 (d, J = 6,7, 3H); 1,13 (d, J = 6,7, 3H); 1,3-1,45 (m, 3H); 1,36 (d, J = 6,4, 3H);1,7-1,82 (m, 1H); 2,07 (s, 3H); 2,35-2,45 (m, 1 H); 2,87 (d, J = 6,9, 1 H); 3-3,28 (m, 4H); 3,54 (d, J = 7,1, 1 H); 4,08 (dd, J = 3,1, J = 10,7, 1 H); 4,19 (d, J = 9,4, 1 H); 4,37-4,47 (m, 2H); 4,5 (d, J = 3,4, 1 H); 4,75-4,8 (m, 2H); 5,25-5,29 (m, 2H); 6,99 (d, J = 8,4, 2H); 7,26-7,39 (m, 5H); 7,63 (d, J = 8,1, 1 H); 7,67-7,76 (m, 4H); 8 (s, 1 H); 8,07-8,12 (m, 3H).

### Exemple 7 : N-Ac-D-2-Nal¹-cyclo(Cys²-Tyr³-D-Trp⁴-N-Ac-Lys⁵-Val⁶-Cys⁷)-Thr⁸-NH₂ (Ac-D-2-Nal¹-cyclo(Cys²-Tyr³-D-Trp⁴-Lys(Ac)⁵-Val⁶-Cys⁷)-Thr⁸-NH₂)

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-Lys(Ac)-OH puis Fmoc-D-Trp-OH puis Fmoc-L-Tyr(tBu)-OH puis Fmoc-L-Cys(Trt)-OH et enfin Ac-β-(2-naphthyl)-D-Ala-OH,

Le composé est cyclisé par la formation d'un pont disulfure, puis clivé de la résine, purifié, et enfin obtenu sous forme d'acétate selon le mode opératoire décrit précédemment pour conduire au composé N-Ac-D-2-Nal¹-cyclo(Cys²-Tyr³-D-Trp⁴-N-Ac-Lys⁵-Val⁶-Cys⁷)-Thr⁸-NH₂.

HPLC : tr = 12,75 min

HRMS (H₂O) m/z = 1202,4800 [M+Na]⁺ (calc, 1202,4779)

¹H NMR (400 Mhz, D₂O/CD₃CN): δ 0,28-0,40 (m, 1H); 0,41-0,58 (m, 1 H); 0,86 (d, J = 6,7, 3H); 0,89 (d, J = 6,7, 3H); 1,13 (d, J = 6,4, 3H); 1,55-1,65 (m, 1H); 1,81 (s, 3H); 1,85 (s, 3H); 2,16-2,22 (m, 1 H); 2,73-2,94 (m, 7H); 3-3,07 (m, 1 H); 3,29 (dd, J = 5,7, J = 13,8, 1 H); 3,89 (dd, J = 3,1, J = 11, 1 H); 4 (d, J = 9,3, 1 H); 4,13-4,22 (m, 2H); 4,24 (d, J = 3,8, 1 H); 4,53 (dd, J = 6,9, J = 8, 1 H); 4,77 (dd, J = 5,7, J = 8,9, 1 H); 5,22 (dd, J = 4,7, J = 9,6, 1 H); 5,29 (dd, J = 7,3, J = 7,9, 1 H); 6,69 (d, J = 8,4, 2H); 6,99-7,11 (m, 5H); 7,36 (d, J = 8, 1H); 7,41-7,49 (m, 4H); 7,73-7,82 (m, 4H),

### Exemple 8 : H-D-2-Nal¹-cyclo(HomoCys²-Tyr³-D-Trp⁴-Lys⁵-Val⁶-Cys⁷)-Thr⁸-NH₂ (H-D-2-Nal¹-cyclo(Hcy²-Tyr³-D-Trp⁴-Lys⁵-Val⁶-Cys⁷)-Thr⁸-NH₂)

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-D-Trp-OH puis Fmoc-L-Tyr(tBu)-OH puis Fmoc-L-HomoCys(Trt)-OH et enfin Boc-β-(2-naphthyl)-D-Ala-OH,

Le composé est cyclisé par la formation d'un pont disulfure, puis clivé de la résine, purifié, et enfin obtenu sous forme d'acétate selon le mode opératoire décrit précédemment pour conduire au composé H-D-2-Nal1-cyclo(HomoCys2-Tyr3-D-Trp4-Lys5-Val6-Cys7)-Thr8-NH2.

HPLC : tr = 10,27 min

HRMS (H₂O) m/z = 1132,4719 [M+Na]⁺ (calc, 1132,4749)

¹H NMR (400 Mhz, D₂O): δ 0,29-0,45 (m, 1 H); 0,46-0,56 (m, 1 H); 0,79 (d, J = 6,7, 3H); 0,82 (d, J = 6,7, 3H); 1,08 (d, J = 6,4, 3H); 1,15-1,63 (m, 5H); 1,92-2,06 (m, 1 H); 2,49-2,62 (m, 2H); 2,65-2,91 (m, 5H); 3,08 (dd, J = 11,1, J = 12,8, 2H); 3,21 (s, 1 H); 3,37 (dd, J = 5,1, J = 13, 2H); 3,74 (d, J = 9,3, 2H); 3,92 (dd, J = 4,1, J = 9,6, 2H); 4,05-4,21 (m, 6H); 4,25 (d, J = 3,9, 2H); 4,42 (dd, J = 6,5, J = 8,8, 2H); 4,56 (dd, J = 5,3, J = 9,3, 2H); 6,68 (d, J = 8,5, 2H); 6,91-7,12 (m, 5H); 7,29-7,44 (m, 5H); 7,62 (s, 1H); 7,75-7,83 (m, 3H).

### Exemple 9 : N-Ac-D-2-Nal¹-cyclo(Cys²-Tyr³-D-Trp⁴-Lys⁵-Val⁶-Cys⁷)-Thr⁸-NH₂ (Ac-D-2-Nal¹-cyclo(Cys²-Tyr³-D-Trp⁴-Lys⁵-Val⁶-Cys⁷)-Thr⁸-NH₂)

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-D-Trp-OH puis Fmoc-L-Tyr(tBu)-OH puis Fmoc-L-Cys(Trt)-OH et enfin Ac-β-(2-naphthyl)-D-Ala-OH,

Le composé est cyclisé par la formation d'un pont disulfure, puis clivé de la résine, purifié, et enfin obtenu sous forme d'acétate selon le mode opératoire décrit précédemment pour conduire au composé N-Ac-D-2-Nal¹-cyclo(Cys²-Tyr³-D-Trp⁴-Lys⁵-Val⁶-Cys⁷)-Thr⁸-NH₂.

HPLC : tr = 10,77 min

HRMS (H₂O) m/z = 1138,4884 [M+H]⁺ (calc, 1138,4854)

¹H NMR (400 Mhz, D₂O): δ 0,29-0,40 (m, 1 H); 0,42-0,58 (m, 1 H); 0,81 (t, J = 6,7, 6H); 1,11 (d, J = 6,3, 3H); 1,1-1,23 (m, 2H); 1,44-1,55 (m, 1 H); 1,9 (s, 3H); 1,95-2,03 (m, 1 H); 2,37-2,45 (m, 2H); 2,47-2,62 (m, 2H); 2,65-2,78 (m, 3H); 2,87-2,99 (m, 2H); 3,07-3,2 (m, 2H); 3,8 (dd, J = 3,5, J = 10,5, 1 H); 3,86 (d, J = 9,1, 1 H); 4,13-4,22 (m, 3H); 4,47 (t, J = 7,4, 1 H); 4,59-4,78 (m, 2H); 6,69 (d, J = 8,4, 2H); 6,94 (d, J = 8,4, 2H); 7,03-7,16 (m, 3H); 7,34-7,46 (m, 5H); 7,63 (s, 1 H); 7,75-7,8 (m, 3H).

### 42. Etude des composés selon l'invention

### 2.1 Activité des composés octapeptidiques sur les récepteurs à la somatostatine

### 2.1.1 Protocole de mesure de l'affinité des peptides sur les récepteurs de la somatostatine

L'affinité des composés de l'invention pour les récepteurs de la Somatostatine est déterminée par la mesure de l'inhibition de la liaison de [¹²⁵I-Tyr11]SRIF-14 à des préparations membranaires de cellules CHO-K1 transfectées.

Les cellules CHO-K1 exprimant de façon stable chacun des sous types de récepteurs de la somatostatine sont collectées avec 0,5 mM d'EDTA et centrifugées à 500 g pendant 5 minutes à 4° C. Le culot est re-suspendu dans tampon phosphate (PBS) et centrifugé à 500 g pendant 5 minutes à 4° C. Le culot est re-suspendu dans du tampon Tris 50 mM à pH 7,4 et centrifugé à 500 g pendant 5 minutes à 4° C. Les cellules sont lysées par sonication et centrifugées à 39 000 g pendant 10 minutes. Le culot est re-suspendu dans du tampon Tris 50 mM à pH 7,4, un aliquote est prélevé pour le dosage de protéines et le reste est centrifugé à 50 000 g pendant 10 minutes. Les membranes obtenues dans ce dernier culot sont stockées à -80° C.

La mesure de l'inhibition compétitive de la liaison de la [¹²⁵I-Tyr11]SRIF-14 (Perkin Elmer) sur chacun des sous types de récepteurs de la somatostatine est effectuée en duplicats dans des plaques 96 puits en polypropylène. Les membranes cellulaires (5 à 20 µg de protéines/puits) sont incubées avec la [¹²⁵I-Tyr11]SRIF-14 (0,05 à 0,1 nM) pendant 50 à 90 minutes à 37° C (conditions dépendantes du sous-type de récepteur) dans un milieu tampon HEPES 50 mM pH 7,4, comprenant 0,2 % d'albumine bovine de sérum (BSA), MgCl2 5 mM, Trasylol 200 KIU/mL, Bacitracine 0,02 mg/mL, phénylméthylsulfonyl fluoride 0,02 mg/mL.

La [¹²⁵I-Tyr11]SRIF-14 liée est séparée de la [¹²⁵I-Tyr11]SRIF-14 libre par filtration à travers des plaques de fibre de verre GF/C (Unifilter, Perkin Elmer) pré-imprégnées avec 0,1 % de polyéthylènimine (P,E,I,), en utilisant un Filtermate 96 (Perkin Elmer). Les filtres sont lavés avec du tampon Tris-HCl 50 mM, pH 7,4 à 4° C et la radioactivité présente est déterminée à l'aide d'un compteur (TopCount, Perkin Elmer).

Les données sont analysées par régression non linéaire assistée par le logiciel XLfit 4,2 (IDBS).

### 2.1.2 Résultats

Les exemples 1, 2, 3, 4, 5, 6, 7, 8 et 9 ont une affinité sur le sous-type 2 des récepteurs à la somatostatine inférieure ou égale à 5 µM.

Les exemples 1, 2, 3, 4, 6, 7, 8 et 9 ont une affinité sur le sous-type 2 des récepteurs à la somatostatine inférieure ou égale à 700 nM.

Les exemples 1, 2, 3, 8 et 9 ont une affinité sur le sous-type 2 des récepteurs à la somatostatine inférieure ou égale à 6 nM.

Les exemples 1, 2, 3, 4, 5, 6, 8 et 9 ont une affinité sur le sous-type 5 des récepteurs à la somatostatine inférieure ou égale à 5 µM.

Les exemples 1, 2, 3, 8 et 9 ont une affinité sur le sous-type 5 des récepteurs à la somatostatine inférieure ou égale à 500 nM.

Les exemples 3, 8 et 9 ont une affinité sur le sous-type 5 des récepteurs à la somatostatine inférieure ou égale à 10 nM.

Les exemples 1, 2, 3, 4, 5, 8 et 9 ont une affinité sur le sous-type 3 des récepteurs à la somatostatine inférieure ou égale à 1 µM.

Les exemples 2, 3, 8 et 9 ont une affinité sur le sous-type 3 des récepteurs à la somatostatine inférieure ou égale à 200 nM.

Les exemples 8 et 9 ont une affinité sur le sous-type 3 des récepteurs à la somatostatine inférieure ou égale à 100 nM.

## Revendications

1. Composé octapeptidique de formule générale (I)
R-AA¹-cyclo(AA²-Tyr³-D-Trp⁴- AA⁵-Val⁶-Cys⁷)-Thr⁸-NH₂ (I)
dans laquelle AA¹ représente un radical d'acide aminé lié au radical R et à l'acide aminé AA² selon la formule 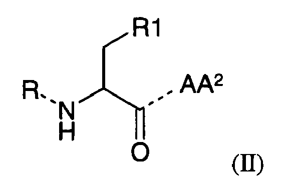 dans laquelle
R1 représente le radical naphthyle éventuellement substitué par un ou plusieurs radicaux alkyle ;
R représente un atome d'hydrogène ou un radicale alkyle ou acétyle ;
AA² représente un radical d'acide aminé lié aux acides aminés AA¹ et Tyr³ selon la formule 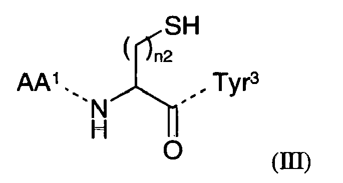 dans laquelle n2 représente un entier de 1 à 2 ;
AA⁵ représente un radical d'acide aminé lié aux acides aminés Trp⁴ et Val⁶ selon la formule 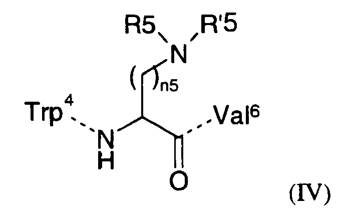 dans laquelle R5 et R'5 représentent indépendamment un atome d'hydrogène ou un radical alkyle ou acétyle, et n5 représente un entier de 1 à 6 ;
étant entendu que
si AA² représente un radical d'acide aminé cystéine, alors
soit AA¹ ne représente pas un radical d'acide aminé naphthylalanine et AA⁵ représente un radical d'acide aminé lysine ;
soit AA¹ représente un radical d'acide aminé naphthylalanine et AA⁵ ne représente pas un radical d'acide aminé lysine ;
soit AA¹ ne représente pas un radical de naphthylalanine et AA⁵ ne représente pas un radical de lysine ;
si AA² ne représente pas un radical de cystéine, alors AA¹ représente un radical d'acide aminé naphthylalanine et AA⁵ représente un radical d'acide aminé lysine,
et étant entendu que tous les acides aminés peuvent être de configuration D ou L,
ou un sel pharmaceutiquement acceptable de ce composé.

2. Composé octapeptidique de formule générale (I) selon la revendication 1, dans laquelle n5 représente un entier de 1 à 4.

3. Composé octapeptidique de formule générale (I) selon l'une des revendications 1 ou 2 dans laquelle AA² représente un radical d'acide aminé cystéine.

4. Composé octapeptidique de formule générale (I) selon l'une des revendications 1 ou 2 dans laquelle AA² ne représente pas un radical d'acide aminé cystéine.

5. Composé octapeptidique de formule générale (I) selon l'une des revendications 1 à 3 dans laquelle AA¹ ne représente pas un radical d'acide aminé naphthylalanine et AA⁵ représente un radical d'acide aminé lysine.

6. Composé octapeptidique de formule générale (I) selon l'une des revendications 1 à 3 dans laquelle AA¹ représente un radical d'acide aminé naphthylalanine et AA⁵ ne représente pas un radical d'acide aminé lysine.

7. Composé octapeptidique de formule générale (I) selon l'une des revendications 1 à 3 dans laquelle AA¹ ne représente pas un radical d'acide aminé naphthylalanine et AA⁵ ne représente pas un radical d'acide aminé lysine.

8. Composé octapeptidique de formule générale (I) selon la revendication 1 ou 2 dans laquelle AA¹ représente le radical d'acide aminé choisi parmi 2-Nal, Ac-2-Nal et CH₃-2-Nal.

9. Composé octapeptidique de formule générale (I) selon la revendication 1 ou 2 dans laquelle AA² représente le radical d'acide aminé choisi parmi Cys et Hcy.

10. Composé octapeptidique de formule générale (I) selon la revendication 1 ou 2 dans laquelle AA⁵ représente le radical d'acide aminé choisi parmi Lys, Lys(Ac), Orn, (CH₃)₂Lys, Dab et Dap.

11. Composé octapeptidique de formule générale (I) selon la revendication 1 ou 2, choisi parmi :
H-D-2-Nal¹-cyclo(Cys²-Tyr³-D-Trp⁴-Lys(Ac)⁵-Val⁶-Cys⁷)-Thr⁸-NH₂
Ac-D-2-Nal¹-cyclo(Cys²-Tyr³-D-Trp⁴-Lys(Ac)⁵-Val⁶-Cys⁷)-Thr⁸-NH₂
H-D-2-Nal¹-cyclo(Cys²-Tyr³-D-Trp⁴-Orn⁵-Val⁶-Cys⁷)-Thr⁸-NH₂
H-D-2-Nal¹-cyclo(Cys²-Tyr³-D-Trp⁴-(Me)₂Lys⁵-Val⁶-Cys⁷)-Thr⁸-NH₂
H-D-2-Nal¹-cyclo(Hcy²-Tyr³-D-Trp⁴-Lys⁵-Val⁶-Cys⁷)-Thr⁸-NH₂
Ac-D-2-Nal¹-cyclo(Cys²-Tyr³-D-Trp⁴-Lys⁵-Val⁶-Cys⁷)-Thr⁸-NH₂
CH₃-D-2-Nal¹-cyclo(Cys²-Tyr³-D-Trp⁴-Lys⁵-Val⁶-Cys⁷)-Thr⁸-NH₂
H-D-2-Nal¹-cyclo(Cys²-Tyr³-D-Trp⁴-Dab⁵-Val⁶-Cys⁷)-Thr⁸-NH₂
H-D-2-Nal¹-cyclo(Cys²-Tyr³-D-Trp⁴-Dap⁵-Val⁶-Cys⁷)-Thr⁸-NH₂
ou un sel pharmaceutiquement acceptable de ce composé.

12. Composé octapeptidique de formule générale (I) selon la revendication 11, choisi parmi :
H-D-2-Nal¹-cyclo(Cys²-Tyr³-D-Trp⁴-Orn⁵-Val⁶-Cys⁷)-Thr⁸-NH₂ ;
H-D-2-Nal¹-cyclo(Cys²-Tyr³-D-Trp⁴-(Me)₂Lys⁵-Val⁶-Cys⁷)-Thr⁸-NH₂ ;
H-D-2-Nal¹-cyclo(Hcy²-Tyr³-D-Trp⁴-Lys⁵-Val⁶-Cys⁷)-Thr⁸-NH₂ ; et
Ac-D-2-Nal¹-cyclo(Cys²-Tyr³-D-Trp⁴-Lys⁵-Val⁶-Cys⁷)-Thr⁸-NH₂ ;
ou un sel pharmaceutiquement acceptable de ce composé.

13. Composé octapeptidique de formule générale (I) selon la revendication 12, de formule H-D-2-Nal¹-cyclo(Hcy²-Tyr³-D-Trp⁴-Lys⁵-Val⁶-Cys⁷)-Thr⁸-NH₂ ou un sel pharmaceutiquement acceptable de ce composé.

14. Médicament comprenant un composé de formule générale (I) selon l'une des revendications 1 à 13.

15. Composition thérapeutique comprenant un composé de formule générale (I) selon l'une des revendications 1 à 13, en tant que principe actif en association avec au moins un excipient pharmaceutiquement acceptable.

16. Utilisation d'un composé octapeptidique de formule générale (I) selon l'une des revendications 1 à 13 pour fabriquer un médicament.

17. Utilisation selon la revendication 16 **caractérisé en ce que** le médicament est destiné à traiter une pathologie choisie parmi les maladies liées à l'hormone de croissance.

## Patentansprüche

1. Octapeptid-Verbindung der allgemeinen Formel (I)
R-AA¹-Cyclo(AA²-Tyr³-D-Trp⁴-AA⁵-Val⁶-Cys⁷)-Thr⁸-NH₂ (I),
in der AA¹ einen Aminosäurerest, der an Rest R und Aminosäure AA² gebunden ist, gemäß der Formel 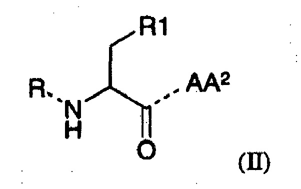 darstellt, in der
R¹ den Naphthyl-Rest darstellt, der gegebenenfalls mit einem oder mehreren Alkyl-Rest(en) substituiert ist;
R ein Wasserstoffatom oder einen Alkyl- oder Acetyl-Rest darstellt;
AA² einen Aminosäurerest, der an die Aminosäuren AA¹ und Tyr³ gebunden ist, gemäß der Formel 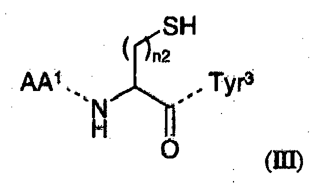 darstellt, in der n2 eine ganze Zahl von 1 bis 2 darstellt;
AA⁵ einen Aminosäurerest, der an die Aminosäuren Trp⁴ und Val⁶ gebunden ist, gemäß der Formel 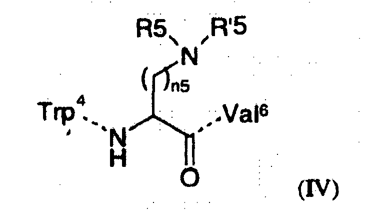 darstellt, in der R5 und R'5 unabhängig ein Wasserstoffatom oder einen Alkyl- oder Acetyl-Rest darstellen und n5 eine ganze Zahl von 1 bis 6 darstellt;
mit der Maßgabe, dass,
wenn AA² einen Cystein-Aminosäurerest darstellt, dann
entweder AA¹ keinen Naphthylalanin-Aminosäurerest darstellt und AA⁵ einen Lysin-Aminosäurerest darstellt;
oder AA¹ einen Naphthylalanin-Aminorest darstellt und AA⁵ keinen Lysin-Aminosäurerest darstellt;
oder AA¹ keinen Naphthylalanin-Rest darstellt und AA⁵ keinen Lysin-Rest darstellt;
wenn AA² keinen Cystein-Rest darstellt, dann AA¹ einen Naphthylalanin-Aminosäurerest darstellt und AA⁵ einen Lysin-Aminosäurerest darstellt,
und mit der Maßgabe, dass alle Aminosäuren D- oder L-Konfiguration haben können,
oder ein pharmazeutisch annehmbares Salz dieser Verbindung.

2. Octapeptid-Verbindung der allgemeinen Formel (I) gemäß Anspruch 1, in der n5 eine ganze Zahl von 1 bis 4 darstellt.

3. Octapeptid-Verbindung der allgemeinen Formel (I) gemäß einem der Ansprüche 1 oder 2, in der AA² einen Cystein-Aminosäurerest darstellt.

4. Octapeptid-Verbindung der allgemeinen Formel (I) gemäß einem der Ansprüche 1 oder 2, in der AA² keinen Cystein-Aminosäurerest darstellt.

5. Octapeptid-Verbindung der allgemeinen Formel (I) gemäß einem der Ansprüche 1 bis 3, in der AA¹ keinen Naphthylalanin-Aminosäurerest darstellt und AA⁵ einen Lysin-Aminosäurerest darstellt.

6. Octapeptid-Verbindung der allgemeinen Formel (I) gemäß einem der Ansprüche 1 bis 3, in der AA¹ einen Naphthylalanin-Aminosäurerest darstellt und AA⁵ keinen Lysin-Aminosäurerest darstellt.

7. Octapeptid-Verbindung der allgemeinen Formel (I) gemäß einem der Ansprüche 1 bis 3, in der AA¹ keinen Naphthylalanin-Aminosäurerest darstellt und AA⁵ keinen Lysin-Aminosäurerest darstellt.

8. Octapeptid-Verbindung der allgemeinen Formel (I) gemäß Anspruch 1 oder 2, in der AA¹ den Aminosäure-Rest darstellt, der aus 2-Nal, Ac-2-Nal und CH₃-2-Nal ausgewählt ist.

9. Octapepdid-Verbindung der allgemeinen Formel (I) gemäß Anspruch 1 oder 2, in der AA² den Aminosäure-Rest darstellt, der aus Cys und Hcy ausgewählt ist.

10. Octapeptid-Verbindung der allgemeinen Formel (I) gemäß Anspruch 1 oder 2, in der AA⁵ den Aminosäure-Rest darstellt, der aus Lys, Lys(Ac), Orn, (CH₃)₂Lys, Dab und Dap ausgewählt ist.

11. Octapeptid-Verbindung der allgemeinen Formel (I) gemäß Anspruch 1 oder 2, ausgewählt aus:
H-D-2-Nal¹-Cyclo(Cys²-Tyr³-D-Trp⁴-Lys(Ac)⁵-Val⁶-Cys⁷)-Thr⁸-NH₂
Ac-D-2-Nal¹-Cyclo(Cys²-Tyr³-D-Trp⁴-Lys(Ac)⁵-Val⁶-Cys⁷)-Thr⁸-NH₂
H-D-2-Na¹-Cyclo(Cys²-Tyr³-D-Trp⁴-Orn⁵-Val⁶-Cys⁷)-Thr⁸-NH₂
H-D-2-Nal¹-Cyclo(Cys²-Tyr³-D-Trp⁴-(Me)₂Lys⁵-Val⁶-Cys⁷)-Thr⁸-NH₂
H-D-2-Nal¹-Cyclo(Hcy²-Tyr³-D-Trp⁴-Lys⁵-Val⁶-Cys⁷)-Thr⁶-NH₂
Ac-D-2-Nal¹-Cyclo(Cys²- Tyr³-O-Trp⁴-Lys⁵-Val⁶-Cys⁷)-Thr⁸-NH₂
CH₃-D-2-Nal¹-Cyclo(Cys²-Tyr³-O-Trp⁴-Lys⁵-Val⁶-Cys⁷)-Thr⁸-NH₂
H-D-2-Nal¹-Cyclo(Cys²-Tyr³-D- Trp⁴-Dab⁵-Val⁶-Cys⁷)-Thr⁸-NH₂
H-D-2-Nal¹-Cyclo(Cys²-Tyr³-D-Trp⁴-Dap⁵-Val⁶-Cys⁷)-Thr⁸-NH₂
oder ein pharmazeutisch annehmbares Salz dieser Verbindung.

12. Octapeptid-Verbindung der allgemeinen Formel (I) gemäß Anspruch 11, ausgewählt aus:
H-D-2-Nal¹-Cyclo(Cys²-Tyr³-D-Trp⁴-Orn⁵-Val⁶-Cys⁷)-Thr⁸-NH₂;
H-D-2-Nal¹-Cyclo(Cys²-Tyr³-D-Trp⁴-(Me)₂Lys⁵-Val⁶-Cys⁷)-Thr⁸-NH₂;
H-D-2-Nal¹-Cyclo(Hcy²-Tyr³-D-Trp⁴-Lys⁵-Val⁶-Cys⁷)-Thr⁸-NH₂ und
Ac-D-2-Nal¹-Cyclo(Cys²-Tyr³-D-Trp⁴-Lys⁵-Val⁶-Cys⁷)-Thr⁸-NH₂;
oder ein pharmazeutisch annehmbares Salz dieser Verbindung.

13. Octapeptid-Verbindung der allgemeinen Formel (I) gemäß Anspruch 12 der Formel
H-D-2-Nal¹-Cyclo(Hcy²-Tyr³-D-Trp⁴-Lys⁵-Val⁶-Cys⁷)-Thr⁸-NH₂
oder ein pharmazeutisch annehmbares Salz dieser Verbindung.

14. Medikament, das eine Verbindung der allgemeinen Formel (I) gemäß einem der Ansprüche 1 bis 13 umfasst.

15. Therapeutische Zusammensetzung, die eine Verbindung der allgemeinen Formel (I) gemäß einem der Ansprüche 1 bis 13 als Wirkstoff in Verbindung mit wenigstens einem pharmazeutisch annehmbaren Exzipiens umfasst.

16. Verwendung einer Octapeptid-Verbindung der allgemeinen Formel (I) gemäß einem der Ansprüche 1 bis 13 zur Herstellung eines Medikaments.

17. Verwendung gemäß Anspruch 16, **dadurch gekennzeichnet, dass** das Medikament dazu bestimmt ist, eine Pathologie, ausgewählt aus Krankheiten, die mit Wachstumshormon verbunden sind, zu behandeln.

## Claims

1. Octapeptide compound of general formula (I)
R-AA¹-cyclo(AA²-Tyr³-D-Trp⁴- AA⁵-Val⁶-Cys⁷)-Thr⁸-NH₂ (I)
in which AA¹ represents an amino acid radical linked to the radical R and to the amino acid AA² according to the formula 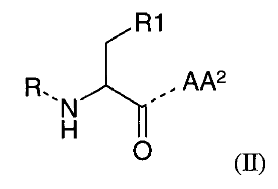 in which
R1 represents the naphthyl radical optionally substituted by one or more alkyl radicals;
R represents a hydrogen atom or an alkyl or acetyl radical;
AA² represents an amino acid radical linked to the amino acids AA¹ and Tyr³ according to the formula 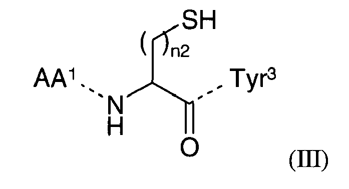 in which n2 represents an integer from 1 to 2;
AA⁵ represents an amino acid radical linked to the amino acids Trp⁴ and Val⁶ according to the formula 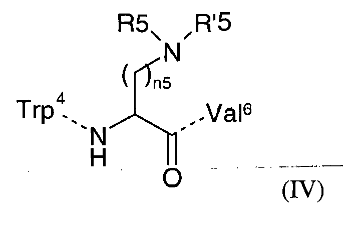 in which R5 and R'5 represent independently a hydrogen atom or an alkyl or acetyl radical, and n5 represents an integer from 1 to 6;
it being understood that
if AA² represents a cysteine amino acid radical, then
either AA¹ does not represent a naphthylalanine amino acid radical and AA⁵ represents a lysine amino acid radical;
or AA¹ represents a naphthylalanine amino acid radical and AA⁵ does not represent a lysine amino acid radical;
or AA¹ does not represent a radical of naphthylalanine and AA⁵ does not represent a lysine radical;
if AA² does not represent a cysteine radical, then AA¹ represents a naphthylalanine amino acid radical and AA⁵ represents a lysine amino acid radical,
and it being understood that all the amino acids can be of D or L configuration,
or a pharmaceutically acceptable salt of this compound.

2. Octapeptide compound of general formula (I) according to claim 1, in which n5 represents an integer from 1 to 4.

3. Octapeptide compound of general formula (I) according to one of claims 1 or 2, in which AA² represents a cysteine amino acid radical.

4. Octapeptide compound of general formula (I) according to one of claims 1 or 2, in which AA² does not represent a cysteine amino acid radical.

5. Octapeptide compound of general formula (I) according to one of claims 1 to 3, in which AA¹ does not represent a naphthylalanine amino acid radical and AA⁵ represents a lysine amino acid radical.

6. Octapeptide compound of general formula (I) according to one of claims 1 to 3, in which AA¹ represents a naphthylalanine amino acid radical and AA⁵ does not represent a lysine amino acid radical.

7. Octapeptide compound of general formula (I) according to one of claims 1 to 3, in which AA¹ does not represent a naphthylalanine amino acid radical and AA⁵ does not represent a lysine amino acid radical.

8. Octapeptide compound of general formula (I) according to claim 1 or 2, in which AA¹ represents the amino acid radical chosen from 2-Nal, Ac-2-Nal and CH₃-2-Nal.

9. Octapeptide compound of general formula (I) according to claim 1 or 2, in which AA² represents the amino acid radical chosen from Cys and Hcy.

10. Octapeptide compound of general formula (I) according to claim 1 or 2, in which AA⁵ represents the amino acid radical chosen from Lys, Lys(Ac), Orn, (CH₃)₂Lys, Dab and Dap.

11. Octapeptide compound of general formula (I) according to claim 1 or 2, chosen from:
H-D-2-Nal¹-cyclo(Cys²-Tyr³-D-Trp⁴-Lys(Ac)⁵-Val⁶-Cys⁷)-Thr⁸-NH₂
Ac-D-2-Nal¹-cyclo(Cys²-Tyr³-D-Trp⁴-Lys(Ac)⁵-Val⁶-Cys⁷)-Thr⁸-NH₂
H-D-2-Nal¹-cyclo(Cys²-Tyr³-D-Trp⁴-Orn⁵-Val⁶-Cys⁷)-Thr⁸-NH₂
H-D-2-Nal¹-cyclo(Cys²-Tyr³-D-Trp⁴-(Me)₂Lys⁵-Val⁶-Cys⁷)-Thr⁸-NH₂
H-D-2-Nal¹-cyclo(Hcy²-Tyr³-D-Trp⁴-Lys⁵-Val⁶-Cys⁷)-Thr⁸-NH₂
Ac-D-2-Nal¹-cyclo(Cys²-Tyr³-D-Trp⁴-Lys⁵-Val⁶-Cys⁷)-Thr⁸-NH₂
CH₃-D-2-Nal¹-cyclo(Cys²-Tyr³-D-Trp⁴-Lys⁵-Val⁶-Cys⁷)-Thr⁸-NH₂
H-D-2-Nal¹-cyclo(Cys²-Tyr³-D-Trp⁴-Dab⁵-Val⁶-Cys⁷)-Thr⁸-NH₂
H-D-2-Nal¹-cyclo(Cys²-Tyr³-D-Trp⁴-Dap⁵-Val⁶-Cys⁷)-Thr⁸-NH₂
or a pharmaceutically acceptable salt of this compound.

12. Octapeptide compound of general formula (I) according to claim 11, chosen from:
H-D-2-Nal¹-cyclo(Cys²-Tyr³-D-Trp⁴-Orn⁵-Val⁵-Cys⁶)-Thr^{B}-NH₂;
H-D-2-Nal¹-cyclo(Cys²-Tyr³-D-Trp⁴-(Me)₂Lys⁵-Val⁶-Cys⁷)-Thr⁸-NH₂;
H-D-2-Nal¹-cyclo(Hcy²-Tyr³-D-Trp⁴-Lys⁵-Val⁶-Cys⁷)-Thr⁸-NH₂; and
Ac-D-2-Nal¹-cyclo(Cys²-Tyr³-D-Trp⁴-Lys⁵-Val⁶-Cys⁷)-Thr⁸-NH₂;
or a pharmaceutically acceptable salt of this compound.

13. Octapeptide compound of general formula (I) according to claim 12, of formula H-D-2-Nal¹-cyclo(Hcy²-Tyr³-D-Trp⁴-Lys⁵-Val⁶-Cys⁷)-Thr⁸-NH₂ or a pharmaceutically acceptable salt of this compound.

14. Medicament comprising a compound of general formula (I) according to one of claims 1 to 13.

15. Therapeutic composition comprising a compound of general formula (I) according to one of claims 1 to 13, as active ingredient in combination with at least one pharmaceutically acceptable excipient.

16. Use of an octapeptide compound of general formula (I) according to one of claims 1 to 13 for producing a medicament.

17. Use according to claim 16, **characterized in that** the medicament is intended to treat a pathology chosen from the diseases linked to the growth hormone.
